# NEUE EUROPÄISCHE PATENTSCHRIFT

(11) **EP 1 553 984 B2**
(45) Veröffentlichungstag und Bekanntmachung des Hinweises auf die Entscheidung über den Einspruch: **30.05.2018**
(45) Hinweis auf die Patenterteilung: 29.10.2008
(21) Anmeldenummer: 03757950.5
(22) Anmeldetag: 10.10.2003
(51) Int. Cl.: A61K 49/00

(54) **VERWENDUNG EINES FÄRBEMITTELS ZUR ANFÄRBUNG DER MEMBRANA LIMITANS**
USE OF A DYE FOR COLOURING THE MEMBRANA LIMITANS
UTILISATION D'UN AGENT DE COLORATION DESTINE A COLORER LA MEMBRANA LIMITANS

(30) Priorität: 14.10.2002 DE 10247781; 28.11.2002 DE 10255601
(43) Veröffentlichungstag der Anmeldung: 20.07.2005
(73) Patentinhaber: FLUORON GMBH, 89077 Ulm (DE)
(72) Erfinder: MEINERT, Hasso, 89231 Neu-Ulm (DE); GÜNTHER, Bernhard, 69221 Dossenheim (DE); KIM, Yong-Keun;, 89231 Neu-Ulm (DE); HIEBL, Wilfried, 89257 Illertissen (DE)
(74) Vertreter: Schiweck, Weinzierl & Koch Patentanwälte Partnerschaft mbB
(86) Internationale Anmeldenummer: PCT/EP2003/011251
(87) Internationale Veröffentlichungsnummer: WO 2004/035091

(56) Entgegenhaltungen:
- EP-A- 0 037 905
- WO-A-02/07693
- WO-A-99/58160
- WO-A-03/043548
- WO-A1-99/58159
- GB-A- 2 026 015
- US-A1- 2003 096 334
- SOLOMON K D ET AL: "Protective effect of the anterior lens capsule during extracapsular cataract extraction" OPHTHALMOLOGY, J. B. LIPPINCOTT CO., PHILADELPHIA, PA, US, Bd. 96, Nr. 5, 1. Mai 1989 (1989-05-01), Seiten 591-597, XP002082494 ISSN: 0161-6420 in der Anmeldung erwähnt
- M. VECKENEER ET AL: "Ocular toxicity study of trypan blue injected into the vitreous cavity of rabbit eyes" GRAEFE'S ARCHIVE OF CLINICAL AND EPERIMENTAL OPHTHALMOLOGY, Bd. 239, Nr. 9, 2001, Seiten 698-704, XP002268940 in der Anmeldung erwähnt
- E. KUTSCHERA: "Vitalfärbung der Abgehobenen Netzhaut und ihrer Defekte" ALBRECHT V. GRAEFES ARCHIVE FOR CLINICAL AND EXPERIMENTAL OPHTHALMOLOGY, Bd. 178, 1969, Seiten 72-87,
- S. MENNEL ET AL: "Patent Blue: A Novel Vital Dye in Vitreoretinal Surgery" OPHTHALMOLOGICA: INTERNATIONAL JOURNAL OF OPHTHALMOLOGY, Bd. 220, Nr. 3, 2006, Seiten 190-193,
- C. LÜKE ET AL: "Retinal tolerance to dyes" BRITISH JOURNAL OF OPHTHALMOLOGY, Bd. 89, 2005, Seiten 1188-1191,
- Blueron Enzigartges kapselfärbemittel mit herausragender Biokompatibilität
- ÜNLÜ K. ET AL: 'Gentian violet solution for staining the anterior capsule' J CATARACT REFRACT SURG Bd. 26, August 2000, Seiten 1228 - 1232
- GAMAL ELDIN S.A. ET AL: 'Experimental staining of the anterior lens capsule in albino rabbits' J CATARACT REFRACT SURG Bd. 25, September 1999, Seiten 1289 - 1294

## Beschreibung

Die Erfindung betrifft die Verwendung einer wässrigen, physiologisch kompatiblen Lösung, in welcher ein biokompatibler Triphenylmethan-Farbstoff gelöst ist zur Herstellung eines Färbemittels zur Anfärbung von der aus dem Auge zu entfernenden Membrana limitans interna bei Netzhaut- und Glaskörperchirurgie, wobei der Farbstoff neben toten Zellen zur Unterscheidung vom lebenden Material auch die lebenden Zellen einfärbt.

Aus WO 02/07693 A sind Zusammensetzungen zur Anfärbung von epithelialen Zellen mittels mitochondrialen Farbstoffen, insbesondere zur Diagnose von kanzerösem oder präkanzerösem epitherialen Gewebes bekannt. Als Farbstoffe können wässrige, gepufferte Lösung von Machalite Green und Brilliant Green zum Einsatz kommen.

Aus GB-A-2,026,015 sind zum Anfärben von Zellen Zusammensetzungen bekannt, welche unter anderem 0,1 bis 0,5 % eines Triphenylmethan-Farbstoffs enthalten, wobei diese Zusammensetzungen als Lösungen hergestellt werden können.

Aus EP-A-0 037 905 ist ein Verfahren zum Nachweis und zur differentialdiagnostischen Bestimmung leukämischer Zellen bekannt, bei dem man einen Triphenylmethan-Farbstoff verwendet.

Ferner ist es aus der WO 99/58160 bekannt, als Farbstoff Trypanblau zu verwenden. Diese aus der Klasse der Diazo-Farbstoffe bekannte Verbindung wird in einer wässrigen Lösung zum Einfärben der Vorderkapsel für eine Katarakt-Operation am Auge verwendet. Durch die Visualisierung der Vorderkapsel erkennt der Chirurg den Umriss der Kapsulorhexis, wodurch die Phakoemulsifikation erleichtert wird.

Bei Trypanblau handelt es sich um eine zytotoxische Substanz, wie beispielsweise aus Solomon K.D. et al.: Protective effect of the anterior lens capsule during extracapsular cataract extraction, OPHTHALMOLOGY, vol. 96, no. 5, May 1989 (1989-05), 591-597 und Veckener M. et al.: Ocular toxicity study of trypan blue injected into the vitreous cavity of rabit eyes, Graefe's Arch Clin Ex Ophthalmol (2002) 239: 698-704 bekannt ist. Bei der Verwendung von Trypanblau ist daher die vollständige Ausspülung insbesondere des Augenbereiches, in welchem das Trypanblau als Färbemittel zum Einsatz gekommen ist, unmittelbar nach der Kataraktoperation erforderlich, um einen längeren Verbleib im Körper bzw. im Auge zu vermeiden.

Aus US-A-4,764,360 ist es bekannt, einem hochmolekularen Polymerisat, welches einen Träger bildet, einen Farbstoff mit einem Molekulargewicht von wenigstens 10.000 zuzufügen.

Hierdurch soll vermieden werden, dass der Farbstoff in das umgebende Körpergewebe eindringt. Der Farbstoff soll nur den hochmolekularen Träger einfärben.

Ferner ist es bekannt (E. Kutchera, "Vitalfärbung der abgehobenen Netzhaut und ihre Defekte", Albrecht v. Graefes Arch. klin. exp. Ophthal. 178, 72,87 (1969)), zur Sichtbarmachung von die gesamte Netzhaut betreffenden Defekten in Fällen von Netzhautabhebung den Farbstoff Patentblau intravitreal zu injizieren. Für die intravitreale Injektion wurde eine 0,47%-ige Patentblau-Hyaluronsäure-Lösung verwendet. Die Visualisierung der Netzhautabhebung ist äußerst zeitaufwendig und ergibt sich erst einige Tage nach der Injektion.

Aufgabe der Erfindung ist es, die Herstellung eines Färbemittels mit fehlender Zytotoxizität zu schaffen, welches zur Sichtbarmachung der Membrana limitans interna im menschlichen oder tierischen Auge geeignet ist.

Diese Aufgabe wird erfindungsgemäß durch die Merkmale des Patentanspruches 1 gelöst. Die Unteransprüche beinhalten vorteilhafte Weiterbildungen der Erfindung.

Bei der Erfindung wird durch die Verwendung eines biokompatiblen Triphenylmethan-Farbstoffs trägerlos in einer physiologisch kompatiblen wässrigen Lösung von insbesondere Natriumchlorid, die mit einem Puffer auf ein pH von 6,8 bis 7,8, insbesondere etwa 7,4 eingestellt sein kann, ein Färbemittel zum Anfärben der Membranen im menschlichen und tierischen Auge geschaffen. Beim Einfärbemittel handelt es sich um einen nichtpolymeren, niedermolekularen, wasserlöslichen Farbstoff. Das bei der Erfindung zur Anwendung kommende Einfärbemittel kann zur Vitalitätsprüfung eingesetzt werden, wobei jedoch im Unterschied zu herkömmlichen Vitalfarbstoffen mit dem bei der Erfindung zur Anwendung kommenden biokompatiblen Farbstoff neben den toten Zellen zur Unterscheidung vom lebenden Material auch die lebenden Zellen eingefärbt werden können.

Als ein wasserlöslicher, niedermolekularer Farbstoff kommt ein Triphenylmethan-Farbstoff zum Einsatz. Der Farbstoff kommt trägerfrei zum Einsatz. Beispiele für derartige geeignete Farbstoffe sind Patentblau und Brillantblau R, wobei letzterer von der Proteinfärbung bei der Gelelektrophorese bekannt ist.

Bei Patentblau handelt es sich vorzugsweise um ein als Lebensmittelfarbstoff (L-Blau 3 = E 131) zugelassenes Patentblau V (C₅₄H₆₂CaN₄O₁₄S₄, MG: 1159,45).

Als Puffer kann ein Phosphat-, Hydrogencarbonat- oder Citrat-Puffer, dessen pH-Wert mittels Natriumhydroxid eingestellt werden kann, verwendet werden. Die Konzentration des biokompatiblen Farbstoffs, z.B. von Patentblau V beträgt in wässriger Lösung vorzugsweise 0,6 bis 2,5 g/l, insbesondere etwa 1,2 g/l. Man erreicht eine spontane Einfärbung der gewünschten Bereiche im menschlichen oder tierischen Körper.

Das Färbemittel kann zum Anfärben der Linsenkapsel, insbesondere der Linsenvorderkapsel bei einer Katarakt-Operation verwendet werden. Die Anfärbung erfolgt vor der Kapsulorhexi.s und Phakoemulsifikation.

Für die Anfärbung wird das Kammerwasser durch eine korneale oder sklerale Tunnelinzision abgesaugt und die Vorderkammer anschließend mit einem Gas, insbesondere Luft gefüllt. Mit einer Kanüle werden ca. 0,3 ml Färbemittellösung, z.B. von Patentblau V in die Vorderkammer verabreicht. Es entsteht die Anfärbung der Linsenkapsel, welche durch den Pupillenrand der Iris begrenzt ist. Nach einigen Sekunden wird zum Auswaschen des nicht benötigten Farbstoffes die Vorderkammer mit einer Natriumchlorid-Lösung ausgespült.

Anschließend wird für die Durchführung der Katarakt-Operation in herkömmlicher Weise eine viskoelastische Lösung in die Augenvorderkammer eingebracht. Aufgrund der blauen Verfärbung der Vorderkapsel tritt der Umriss der Kapsulorhexis klar hervor und lässt sich vom grauen Gewebe des Linsenkerns klar unterscheiden.

Ferner kann das Färbemittel zum Anfärben der Membrana limitans interna oder beispielsweise infolge von PVR (proliferative Vitreoretinopathie) entstandenen Membranen, insbesondere epiretinalen Membranen an der Netzhaut oder an der Rückfläche der Glaskörpergrenzmembran, insbesondere bei Netzhaut- und Glaskörperchirurgie zum Einsatz kommen.

Beim Entfernen, beispielsweise einer epiretinalen Membran von der Netzhaut wird mit Hilfe einer über die Pars plana eingebenden Kanüle der Farbstoff, beispielsweise Patentblau V in ca. 0,3 ml der angegebenen Pufferlösung selektiv zur zu entfernenden Membran gebracht. Der Glaskörper kann vorher ganz oder teilweise durch eine Gasfüllung, wie sie in herkömmlicher Weise bei der Glaskörper- oder Netzhaut-, insbesondere Makulachirurgie zum Einsatz kommt, ersetzt sein. Beim Anfärben der epiretinalen Membran kann gegebenenfalls eine Anfärbung des benachbarten Retinagewebes mit schwächerem Einfärbungsgrad erfolgen. Beim Entfernen der Membran von dem darunter liegenden, nichteingefärbten Retinagewebe ergibt sich dann ein guter Kontrast. Nach dem Anfärben wird überschüssige Färbemittellösung ausgespült und der freie Raum durch den angesprochenen gasförmigen Glaskörperersatz angefüllt. Durch die Einfärbung ist es möglich, mit einem nicht beleuchteten oder nur schwach beleuchteten Instrumenten beim Abtragen der Membran zu arbeiten. Hierdurch wird bei ausreichender Kontrastwahrnehmung die Lichttoxizität erheblich verringert. Insbesondere bei der Anwendung im Zusammenhang mit epiretinaler Membranen (Epiretinale Gliose, "macular pucker", "surface wrinkling") bildet der Einsatz der Färbemittellösung eine wertvolle Hilfe beim Aufsuchen und Entfernen der Membranen.

Wenn bei Makulaforamen mit zunehmender Lochgröße die Entfernung der Membrana limitans interna erforderlich ist, erweist sich die Einfärbung dieser Membran mit der Färbemittellösung als vorteilhaftes Hilfsmittel beim Aufsuchen und Entfernen dieser Membran während der Glaskörperchirurgie.

Ferner ist es möglich, ein viskoelastisches Material, beispielsweise Hyaluronsäure, welches als Hilfsmittel bei der ophthalmologischen Chirurgie zum Einsatz kommt, mit der wässrigen Färbemittellösung einzufärben. Insbesondere lässt sich hierdurch bei der Katarakt-Operation eine Verbesserung des Kontrastes des viskoelastischen Hilfsmittels gegenüber dem intraokularen Gewebe, insbesondere der Augeniris und dem Fundusreflex erzielen.

Gegenüber dem herkömmlichen Trypanblau, welches teratogene oder mutagene Wirkung (Cahen RL: Evaluation of the teratogenicity of drugs, Clin Pharmacol. Ther, 1964, 5, 480-514 und Produktinformation BLURHEX™, Dr. Agarwal's Pharma Ltd. Chennai (Indien)) hat, besitzt die erfindungsgemäße biokompatible Lösung, beispielsweise von Patentblau V oder Brillantblau R keine Zytotoxizität.

Zum Nachweis fehlender Zytotoxität wurden Mauszellen L 929 und ARPE-19-Zellen mit dem erfindungsgemäßen Färbemittel Patentblau V mit unterschiedlichen Konzentrationen über 68 bis 72 Stunden im Brutschrank behandelt. Die Vitalität der Zellen und eine abgeleitete Zytotoxizität wird durch Bestimmung des Proteingehalts der behandelten Zellkulturen gegenüber unbehandelten Kontrollkulturen quantitativ bestimmt. Mit einem Standard-Verfahren wird der Proteingehalt kolorimetrisch ermittelt.

Dabei zeigt sich, dass eine Zytotoxizität in signifikanter Höhe entsprechend einer Wachstumhemmung von mehr als 30% nicht vorhanden ist.

Die Erfindung erweist sich insbesondere von Vorteil bei der Durchführung von Katarakt-Operationen mit dichten Katarakten und/oder schwer pigmentierten Fundi, bei denen der Fundusreflex nur schwach ausgebildet ist oder fehlt. Mit Hilfe des Einfärbemittels erreicht man einen guten Kontrast zwischen der gefärbten Vorderkapsel und dem unterliegenden Gewebe.

Im folgenden werden Ausführungsbeispiele des Färbemittels in verschiedenen Pufferlösungen angegeben.

### Beispiel 1

Patentblau V mit einer Konzentration von 1,2 g/l in einer Phosphat-Puffer-Lösung.
200 ml Lösung enthalten:
0,240 g Patentblau V
0,380 g Dinatriumhydrogenphosphat (Na₂HPO₄ × 2 H₂O)
0,060 g Natriumdihydrogenphosphat (NaH₂PO₄ × 2 H₂O)
1,640 g Natriumchlorid (NaCl)
Natriumhydroxid zur pH-Einstellung.

### Beispiel 2

Patentblau V mit einer Konzentration von 1,2 g/l in einer Hydrogencarbonat-Puffer-Lösung.
200 ml Lösung enthalten:
0,240 g Patentblau V
0,420 g Natriumhydrogencarbonat (NaHCO₃)
1,640 g Natriumchlorid (NaCl)
Natriumhydroxid zur pH-Einstellung

### Beispiel 3

Patentblau V mit einer Konzentration von 1,2 g/l in einer Citrat-Puffer-Lösung.
200 ml Lösung enthalten:
0,240 g Patentblau V
0,216 g Trinatriumcitrat (C₆H₅Na₃O₇ × 2 H₂O)
1,640 g Natriumchlorid (NaCl)
Natriumhydroxid zur pH-Einstellung

Identische Ausführungsbeispiele gemäß Beispiel 1, 2 und 3 können auch mit Brillantblau R mit einer Konzentration von 1,2 g/l hergestellt werden.

Vorzugsweise wird bei den Pufferlösungen der pH-Wert durch Natriumhydroxid eingestellt. Es ist jedoch auch möglich, die Lösung selbst auf den gewünschten pH-Wert (neutral, schwach sauer, schwach alkalisch) innerhalb des bevorzugten Bereiches von 6,8 bis 7,8 einzustellen. Die Einstellung der Konzentration von Patentblau auf vorzugsweise 0,6 bis 2,5 g/l, insbesondere etwa 1,2 g/l erfolgt durch eine entsprechende Menge an Patentblau V.

## Patentansprüche

1. Verwendung einer wässrigen, physiologisch kompatiblen Lösung, in welcher ein biokompatibler Triphenylmethan-Farbstoff, gelöst ist, zur Herstellung eines Färbemittels zur Anfärbung von der aus dem Auge zu entfernenden Membrana limitans interna bei Netzhaut- oder Glaskörperchirurgie, wobei der Farbstoff neben toten Zellen zur Unterscheidung vom lebenden Material auch die lebenden Zellen einfärbt.

2. Verwendung nach Anspruch 1, bei welcher der Farbstoff in einem neutralen oder schwach sauren oder schwach alkalischen Puffer gelöst wird.

3. Verwendung nach Anspruch 1, wobei der Farbstoff in einem Puffer mit einem pH Wert von 6,8 bis 7,8 gelöst wird.

4. Verwendung nach einem der Ansprüche 1 bis 3, wobei ein Phosphat-, Hydrogencarbonat- oder Citrat-Puffer verwendet wird.

5. Verwendung nach einem der Ansprüche 1 bis 4, wobei die Konzentration des Farbstoffs in der Pufferlösung 0,3 bis 2,5 g/l, insbesondere etwa 1,2 g/l beträgt.

6. Verwendung nach einem der Ansprüche 1 bis 5, wobei als Farbstoff Patentblau V verwendet wird.

7. Verwendung nach einem der Ansprüche 1 bis 6, wobei als Farbstoff Brillantblau R verwendet wird.

## Claims

1. Use of an aqueous, physiologically compatible solution in which a biocompatible triphenylmethane dye is dissolved, for the production of a dyeing agent for colouring the *Membrana limitans interna* to be removed from the eye in retinal or vitreous humor surgery, wherein the dye in addition to dead cells to distinguish living material also colours living cells.

2. The use according to claim 1, wherein the dye is dissolved in a neutral or weakly acid or weakly alkaline buffer.

3. The use according to claim 1, wherein the dye is dissolved in a buffer with a pH value of 6.8 to 7.8.

4. The use according to any one of claims 1 to 3, wherein a phosphate, a hydrogen carbonate or a citrate buffer is used.

5. The use according to any one of claims 1 to 4, wherein the concentration of the dye in the buffer solution is 0.3 to 2.5 g/l, in particular approx. 1.2 g/l.

6. The use according to any one of claims 1 to 5, wherein the dye used is patent blue V.

7. The use according to any one of claims 1 to 6, wherein the dye used is brilliant blue R.

## Revendications

1. Utilisation d'une solution aqueuse, physiologiquement compatible, dans laquelle est dissoute un agent de coloration biocompatible à base de triphénylméthane, pour la préparation d'un agent de coloration destiné à colorer la *membrana limitans interna* à retirer de l'oeil dans la chirurgie de la rétine ou du corps vitré, l'agent de coloration colorant outre des cellules mortes aussi les cellules vivantes pour distinguer du matériau vivant.

2. Utilisation selon la revendication 1, dans laquelle l'agent de coloration est dissous dans un tampon neutre ou faiblement acide ou faiblement alcalin.

3. Utilisation selon la revendication 1, dans laquelle l'agent de coloration est dissous dans un tampon d'une valeur pH de 6,8 à 7,8.

4. Utilisation selon l'une quelconque des revendications 1 à 3, dans laquelle un tampon à base de phosphate, d'hydrogénocarbonate ou de citrate est utilisé.

5. Utilisation selon l'une quelconque des revendications 1 à 4, dans laquelle la concentration de l'agent de coloration dans la solution tampon est de 0,3 à 2,5 g/l, surtout d'environ 1,2 g/l.

6. Utilisation selon l'une quelconque des revendications 1 à 5, dans laquelle le bleu patenté V est utilisé en tant qu'agent de coloration.

7. Utilisation selon l'une quelconque des revendications 1 à 6, dans laquelle le bleu brillant R est utilisé en tant qu'agent de coloration.
